# EUROPEAN PATENT SPECIFICATION

(11) **EP 2 934 291 B1**
(45) Date of publication and mention of the grant of the patent: **26.10.2022**
(21) Application number: 13830048.8
(22) Date of filing: 17.12.2013
(51) Int. Cl.: A61B 5/00

(54) **DENTAL APPARATUS AND METHOD OF UTILIZING THE SAME**
ZAHNÄRZTLICHE VORRICHTUNG UND VERFAHREN ZUR VERWENDUNG DAVON
APPAREIL DENTAIRE ET SON PROCÉDÉ D'UTILISATION

(30) Priority: 19.12.2012 US 201261739415 P
(43) Date of publication of application: 28.10.2015
(73) Proprietor: Koninklijke Philips N.V., 5656 AG Eindhoven (NL)
(72) Inventor: VERMEULEN, Olaf Thomas Johan Antonie, NL-5656 AE Eindhoven (NL); DEANE, Steven Charles, NL-5656 AE Eindhoven (NL)
(74) Representative: Philips Intellectual Property & Standards
(86) International application number: PCT/IB2013/061019
(87) International publication number: WO 2014/097135

(56) References cited:
- JP-A- 2004 089 239
- US-A- 5 382 163
- US-A1- 2011 314 618
- FERRETTI DE OLIVEIRA F ET AL: "Time-resolved fluorescence spectroscopy of white-spot caries in human enamel", APPLIED OPTICS, OPTICAL SOCIETY OF AMERICA, WASHINGTON, DC; US, vol. 49, no. 12, 20 April 2010 (2010-04-20), pages 2244-2249, XP001553177, ISSN: 0003-6935
- HANS C GERRITSEN ET AL: "Fluorescence lifetime imaging of oxygen in dental biofilm Invited Paper", PROCEEDINGS OF SPIE, vol. 4164, 1 January 2000 (2000-01-01), XP055085636,
- HEINRICH-WELTZIEN R ET AL: "QUANTITATIVE LIGHT-INDUCED FLUORESCENCE (QLF)-A POTENTIAL METHOD FOR THE DENTAL PRACTITIONER", QUINTESSENCE INTERNATIONAL, QUINTESSENZ VERLAG, BERLIN, DE, vol. 34, no. 3, 1 March 2003 (2003-03-01), pages 181-188, XP008040809, ISSN: 0033-6572

## Description

### BACKGROUND

### Technical Field

The present disclosure relates to a dental apparatus and method of utilizing the same. More particularly, the present disclosure relates to a dental apparatus and method of use of the dental apparatus utilizing a frequency modulated excitation signal causing an emitted fluorescence light that is analyzed to detect dental plaque.

### Description of Related Art

US 5 382 163 A discloses a method and apparatus for detecting dental plaque or calculus deposits on a tooth site using a pulse of broad band light to illuminate the tooth site thereby exciting the deposit to luminescence.

It is desirable to detect plaque deposits in the oral cavity to direct action for removal, for example by using toothbrushes (manual or power), tooth floss, toothpicks, or oral irrigators, as detection indicates the areas at which dental cleaning effort should be focused. Such deposits can be difficult to detect in situ/in vivo on the teeth, gums, tongue, or cheek. It is especially important to detect dental plaque. For detection of dental plaque, it is known to use fluorescence measurement, in which incident radiation is directed at the surfaces of the oral cavity, and fluorescence radiation having characteristics associated with the presence of biological deposits is emitted from the surfaces and is detected.

In the state of the art there are two general methods for detecting dental plaque. One method uses primary fluorescence, where the fluorescence of dental plaque or other dental material itself is monitored. The other method uses secondary fluorescence, where surfaces in the oral cavity suspected of bearing dental plaque are treated with a fluorescent label material which preferentially binds to dental plaque, and the fluorescence emission of the label material on the oral cavity surfaces to which it has bound is detected to indicate the presence of dental plaque.

In accordance with the foregoing, apparatuses configured for detecting dental plaque sometimes utilizes monochromatic light to illuminate a potential dental plaque site. In certain instances, the site may be illuminated by a light having a predetermined wavelength or range. Other methods and/or apparatuses may utilize a fast excitation pulse (e.g., nanosecond or faster) and fast and sensitive detection devices that are enabled (e.g., gated) at very short time intervals after the excitation pulse. Such methods and/or apparatuses, typically, utilize photomultiplier tubes, avalanche photodiodes and/or Kerr-gates.

While the aforementioned methods and apparatuses are suitable for detecting dental plaque, such methods and apparatuses are generally expensive and include components that are, typically, bulky and require high voltages.

### SUMMARY

As can be appreciated, a dental apparatus and method of use of the dental apparatus utilizing a frequency modulated excitation signal causing an emitted fluorescence light that is analyzed to detect dental plaque may prove useful in dentistry. The invention is defined by the independent claims relating to an apparatus and method for detecting dental plaque. The dependent claims define advantageous embodiments.

### BRIEF DESCRIPTION OF THE DRAWINGS

The aspects of the present disclosure may be better understood with reference to the following figures. The components in the figures are not necessarily to scale, emphasis instead being placed upon clearly illustrating the principles of the disclosure. Moreover, in the figures, like reference numerals designate corresponding parts throughout the several views.

In the figures:
Figs. 1A and 1B are front and side views, respectively, of a dental apparatus according to an embodiment of the instant disclosure;
Fig. 2 is block diagram illustrating a controller and subsystem of the dental apparatus shown in Figs. 1A and 1B;
Fig. 3 is block diagram illustrating a controller and subsystem of a dental apparatus according to another embodiment of the instant disclosure;
Fig. 4 is a flow chart illustrating a method of detecting dental plaque;
Fig. 5 is a graph of measurements taken from different samples of teeth illustrating variability of tooth enamel associated with the different samples and demineralized human enamel;
Fig. 6 is a graph of sound enamel and demineralized enamel showing the difference vector between both at a modulation frequency of 45 MHz;
Fig. 7 is a graph of the difference vector between sound enamel and demineralized enamel; and
Fig. 8 is a flow chart illustrating a method of detecting demineralization and/or dental caries according to an embodiment of the present disclosure.

### DETAILED DESCRIPTION

The present disclosure describes various embodiments of apparatuses and methods that utilize one or more power levels of excitation light for detecting one or more tooth anomalies such that the tooth anomalies may be removed. Specifically, a dental apparatus, e.g. an electric toothbrush, is configured to provide a frequency modulated excitation light that is configured to cause an emitted fluorescence light to be reflected back to the dental apparatus for analyzing one or more parameters of the emitted fluorescence that correspond to a decay time of the emitted fluorescence light. The one or more parameters are, subsequently, utilized to detect the presence of dental plaque, and optionally also tooth enamel demineralization, dental caries, etc. When the dental plaque is detected, the dental apparatus may then be utilized to remove the dental plaque.

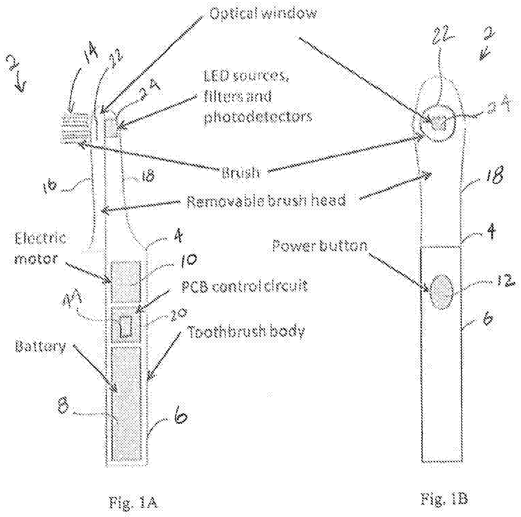

Fig. 1A illustrates a system 2 that is configured to detect dental plaque. System 2 may be configured for use with a variety of handheld dental implements. In the illustrated embodiment, system 2 is in the form of a multipurpose dental apparatus 4 (e.g., a combination electric toothbrush and dental plaque detector). Dental apparatus 4 includes a handle 6 of suitable configuration that is configured to house a battery 8 and an electric motor 10. A power button or switch 12 (Fig. 1B) is provided on the handle 6 and operably couples to battery 8 for supplying power to dental apparatus 4 and components operably associated therewith, e.g., electric motor 10, a controller 20, etc., when depressed. A plurality of bristles 14 of suitable configuration is provided on toothbrush assembly 16 that is configured to detachably couple via one or more coupling methods, e.g., clips (not explicitly shown), to a shaft 18 that extends distally from handle 6.

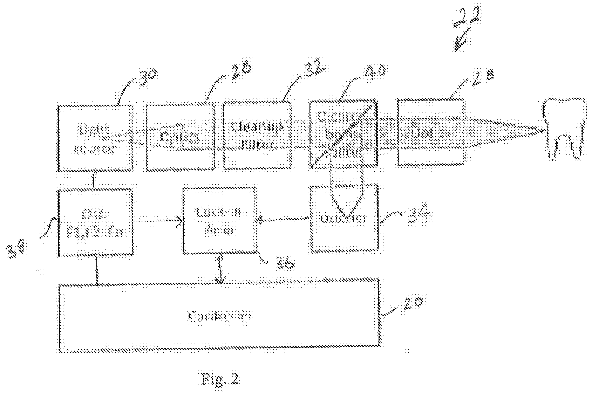

Fig. 2 is block diagram illustrating an embodiment of a controller 20 and subsystem 22 that may be provided with dental apparatus 4. Subsystem 22 may include any suitable electrical and/or non-electrical components that are capable of generating, emitting and/or detecting various power intensities and wavelengths of frequency modulated excitation light, e.g. blue light, red light etc. Subsystem 22 may include, without limitation, for example, one or more light sources 30, photodetectors 34, lock-in amplifier 36, oscillators 38, beam splitters 40, and optical components 28.

Light sources 30 may be any suitable light source. In the embodiment illustrated in Fig. 2, light source 30 is in the form of one or more light emitting diodes 30, e.g., a plurality of light emitting diodes 30 (LEDs 30). LEDs 30 may be configured to generate or emit one or more suitable wavelengths of light. In accordance with the instant disclosure, for example, it has been found that light having wavelengths of 405 nm, 440 nm, 470 nm and/or 480 nm (all visible light, e.g., blue light) were suitable for the purposes described herein. Specifically, light in the visible light spectrum was chosen due to the safety concerns of using other wavelengths of light, e.g., ultraviolet light, infrared, etc., in the mouth, and the cost typically attributed with utilizing such wavelengths of light. Other light sources, e.g. diode laser, and different wavelengths may also be utilized.

Photodetectors 34 are configured to detect the presence of dental plaque on tooth enamel. Specifically, photodetectors 34 are configured and utilized to detect emitted fluorescence associated with tooth material and/or with dental plaque and convert detected emitted fluorescence photons into an electrical signal that is sent to a controller 20 for processing, described in greater detail below.

In accordance with the instant disclosure, photodetectors 34 may be configured to detect emitted fluorescence associated with tooth material and/or with dental plaque in a frequency that ranges from about 10 Hz to about 10 GHz. In one particular embodiment, for example, the frequency may range from about 1 MHz to about 10 GHz. In another embodiment, the frequency may range from about 10Hz to about 100MHz; this particular embodiment may be utilized to optimize signal to noise related to emitted fluorescence decay characteristics of tooth material. Alternatively, in embodiments, image sensors (not explicitly shown) may be utilized in place of photodetectors 34. In this particular embodiment, image processing may be utilized to convert pixel intensities into a form that can be used to determine when dental plaque has been detected. While photodetectors 34 and the image sensors are both suitable for detecting the aforementioned emitted fluorescence associated with tooth material and/or dental plaque, the simplicity of photodetectors 34 makes them ideal for the purposes described herein. As can be appreciated, dental apparatus 4 may include a combination of photodetectors 34 and imaging sensors.

In order to shield photodetectors 34 from various wavelengths of excitation light and/or unwanted background radiation, optical filters (not explicitly shown) may be mounted onto photodetectors 34. Additionally, the photodetectors 34 may include one or more collection and focusing optics such as, for example, lenses, compound parabolic concentrators or a combination of both.

Beam-splitter 40 is utilized to direct frequency modulated excitation light towards teeth and reflect the emitted fluorescence light, which has a longer wavelength, towards photodetectors 34. Accordingly, a dichroic beam splitter which has a short-pass characteristic was utilized for this purpose. Alternatively, instead of using beam splitter 40, we can use two optical paths, one for excitation and one for detection; this may be advantageous in certain embodiments, e.g., to accommodate design variations of dental apparatus 4.

An optional filter 32 may be provided and utilized to block any undesired wavelengths (e.g. ultraviolet light) of light from reaching the teeth or the photodetectors 34. In the illustrated embodiments, for example, filter 32 is a narrow bandpass filter. Filter 32 may be arranged in other configurations to accommodate different wavelengths and/or power intensities of light and/or to achieve different filtering outcomes.

Oscillator 38 may be any suitable oscillator. In the illustrated embodiment, oscillator 38 is operably coupled to LEDs 30 and configured to drive LEDs 30 such that LEDs 30 generate or emit a frequency modulated excitation light or signal. The frequency modulated excitation signal may be emitted at a single frequency or multi-frequencies simultaneously; the latter may be implemented as multiple discrete frequencies (or mixed together) and used to discriminate against potentially interfering materials in a mouth of a user, e.g., composite fillings.

Lock-in amplifier 36 is configured to receive input signals from oscillator 38 and photodetectors 34 and outputs a corresponding signal that corresponds to the detected emitted fluorescence to controller 20. Specifically, oscillator 38 provides a reference frequency (e.g., the frequency of the frequency modulated excitation signal) to lock-in amplifier 36. Lock-in amplifier 36 utilizes this reference frequency to filter out the unwanted portions, e.g., noise, of the signal received from photodetectors 34 and communicates the remaining portions of the signal that is required for processing to controller 20. In essence, lock-in amplifier 36 functions as a phase sensitive detector. As can be appreciated, other amplifiers and/or signal processing devices may be utilized for the purposes described herein.

Subsystem 22 is configured to illuminate tooth material (and in some instances gums) from the frequency modulate excitation signal emitted through toothbrush assembly 16 adjacent to where plurality of bristles 14 are disposed. With this purpose in mind, an optical window 24 (Figs. 1A and 1B) of suitable of configuration is provided on toothbrush assembly 16 adjacent plurality of bristles 14 and is configured to allow light to pass therethrough for detection thereof by subsystem 22. Specifically, when toothbrush assembly 16 is coupled to shaft 18, window 24 aligns with subsystem 22 including LEDs 30, photodetectors 34, amplifiers 36, oscillators 38, beam splitters 40, and optical components 28 such that the frequency modulated excitation signal generated from LEDs 30 is emitted through window 24 and reflected light (e.g., emitted fluorescence of tooth material and/or dental plaque) is transmitted back through window 24 and detected by photodetectors 34.

With reference again to Fig. 1A, dental apparatus 4 includes controller 20 (e.g., a microprocessor) that communicates with subsystem 22 (as best seen in Fig. 2) that is configured to generate, emit and detect light, e.g., frequency modulated excitation signal and emitted fluorescence associated with tooth material and dental plaque. Controller 20 can be a processor, microcontroller, a system on chip (SOC), field programmable gate array (FPGA), etc. Collectively the one or more components, which can include a processor, microcontroller, SOC, and/or FPGA, for performing the various functions and operations described herein are part of a controller 20, as recited, for example, in the claims. Controller 20 can be provided as a single integrated circuit (IC) chip which can be mounted on a single printed circuit board (PCB). Alternatively, the various circuit components of controller 20, including, for example, the processor, microcontroller, etc. are provided as one or more integrated circuit chips. That is, the various circuit components may be located on one or more integrated circuit chips.

Controller 20 communicates with subsystem 22 and is configured to analyze one or more properties of the emitted fluorescence light. In the illustrated embodiment, the one or more properties of the emitted fluorescence light may be phase shift associated with the emitted fluorescence light and/or amplitude of the emitted fluorescence light.

In accordance with the instant disclosure, the phase shift (and/or amplitude) is correlated to a decay time of the emitted fluorescence light and utilized to detect the presence of dental plaque. Specifically, through empirical testing it has been found that there is a direct correlation between a phase shift (and/or amplitude) of detected emitted fluorescence light and fluorescence decay times. More specifically, it is known that emitted fluorescence from a tooth without dental plaque on a surface thereof decays more slowly when compared to emitted fluorescence from a tooth with dental plaque, i.e., a tooth with dental plaque decays faster. Accordingly, detected emitted fluorescence of a tooth with dental plaque will have a lower phase shift at a specific frequency range when compared to a phase shift of detected fluorescence of a tooth without dental plaque. At low frequencies, for example, the phase shift will be zero for both cases, e.g., tooth with/without dental plaque. At very high frequencies both situations will show 90 degrees of phase shift. At frequencies between these two extremes, the phase shift of clean enamel/dentine will be greater than that of the enamel/dentine covered with plaque. In other words: A faster decay time (time domain) means a lower phase shift in the frequency domain. A similar correlation can be made with respect to amplitude of the detected emitted fluorescence. The demodulation of the fluorescence light of a clean enamel/dentin site will be greater at a specific modulation frequency than the demodulation of the fluorescence light of the same site covered with dental plaque. In accordance with the foregoing, controller 20 includes one or more control algorithms that are configured to analyze a phase shift (and/or amplitude) of the emitted fluorescence light utilizing time domain and/or frequency domain analysis methods. The control algorithm may utilize one or more transforms to calculate the phase shift (and/or amplitude). For example, Discrete Fourier Transform (DFT), Fast Fourier Transform (FFT) and/or Laplace Transform may be utilized to calculate the phase shift and/or amplitude of the detected emitted fluorescence.

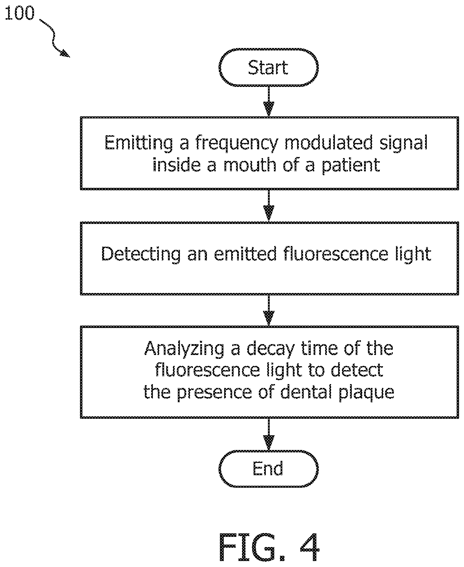

Operation of system 2 is described in terms of a method 100 for detecting dental plaque in the frequency domain. Dental apparatus 4 may, initially, be positioned within a mouth of a user. When the dental apparatus 4 is powered on, plurality of bristles 14 are rotated, in conventional fashion, and a frequency modulated excitation signal is emitted from LEDs 30 (see Fig. 4 at step 102) which causes an emitted fluorescence light to be reflected back to photodetectors 34 (see Fig. 4 at step 104).

Thereafter, controller 20 receives an output signal from lock-in amplifier 36. The control algorithm utilizes one or more of the aforementioned transforms to calculate the phase shift (and/or amplitude) of the detected emitted fluorescence. Controller 20 utilizes a closed loop feedback loop to continuously monitor the presence of a phase shift of the detected emitted fluorescence to ensure that all dental plaque is removed from a tooth site. In embodiments, dental apparatus 4 may be equipped with one or more indicating devices, e.g., audio, visual, etc. (not explicitly shown), that are configured to give a user an indication when a specific site on the tooth is clean. After such an indication, a user can then move to a different site on the tooth or the next tooth. As can be appreciated, this may reduce the overall brushing time of user and/or may also lead to a better, more conscious brushing routine.

The aforementioned process repeats to continuously measure a level of dental plaque on the current tooth being brushed. Dental apparatus 4 can communicate the presence of dental plaque to the user in a wide variety of ways e.g. by illuminating one more LEDS on handle 8 (not explicitly shown).

Dental apparatus 4 helps a user clean their teeth while informing the user if they are removing dental plaque from their teeth and if they have fully removed the dental plaque. Moreover, dental apparatus 4 provides information regarding dental plaque in real time during brushing. Dental apparatus 4 accomplishes the foregoing, without the use of the aforementioned bulky, expensive components that utilize high voltage and that are typically associated with convention dental plaque apparatuses.

From the foregoing and with reference to the various figure drawings, those skilled in the art will appreciate that certain modifications can also be made to the present disclosure without departing from the scope of the same. For example, in some embodiments, subsystem 22 may be built into handle 8 to allow shaft 18 to be replaced. In this instance, frequency modulated excitation signals may be delivered to toothbrush assembly 16 and emitted fluorescence light reflected back to photodetectors 34 and controller 20 via optical fibers (not explicitly shown). Moreover, in this embodiment, one or more light guides (not explicitly shown) may be provided on dental apparatus 4 and configured to channel light to and from window 24.

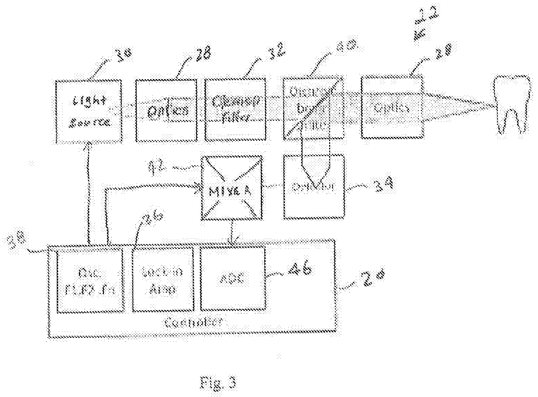

In the embodiment illustrated in Fig. 2, controller 20 and subsystem 22 are configured to function in an analog domain. In embodiments (see Fig. 3 for example), however, oscillator 38 and/or lock-in amplifier 36 can be implemented in a digital domain. In this particular embodiment, digital implementation may be included into controller 20, e.g., an analog heterodyning stage, to down convert signals to an intermediate frequency band better suited for analog-to-digital conversion. Specifically, for each frequency modulated excitation signal, oscillator 38 (a digital oscillator for example) generates a frequency modulated excitation signal with a small offset for a mixer 42 that outputs fall in a frequency range of an analog-to-digital converter 46. Thereafter, all remaining signal processing is done digitally and in accordance with the above.

Moreover, in the instance where a large number of fluorescence measurements are needed, oscillator 38 and LEDs 30 may be configured to generate and emit a maximum length sequence, which would allow detection of fluorescence over a large number of frequencies simultaneously. In this instance, controller 20 may include one or more analog to digital converters 46 and a suitable digital processing unit (not explicitly shown) configured to extract the separate frequency responses and/or transform the separate frequency responses into an impulse response.

Further, while the aforementioned dental apparatus 4 has been described utilizing frequency domain calculations to achieve the above-referenced outcomes, it is within the purview of the instant disclosure to utilize time domain calculations to achieve the above referenced outcomes. As can be appreciated in this instance, one or more modifications may be made to dental apparatus 4 and/or components operably associated therewith. For example, in this particular embodiment, light-source(s) 30 may be pulsed.

As is known, there is a certain variation in lifetime decay data of human enamel. Although the aforementioned dental apparatus 4 does not rely on determination of the actual decay-times, variations of actual decay-times may limit the useful signal range. Accordingly, it may prove useful to calibrate dental apparatus 4 on a clean piece of tooth material for each user individual. To this end, a calibration module 44 (Fig, 1A) may be in operable communication with controller 20 and configured to calibrate dental apparatus 4. As can be appreciated, this may maximize a signal range. Specifically, by calibrating out all phase delays and frequency dependent gains on a clean tooth, the aforementioned analysis will provide a more accurate measure of the amount of dental plaque detected.

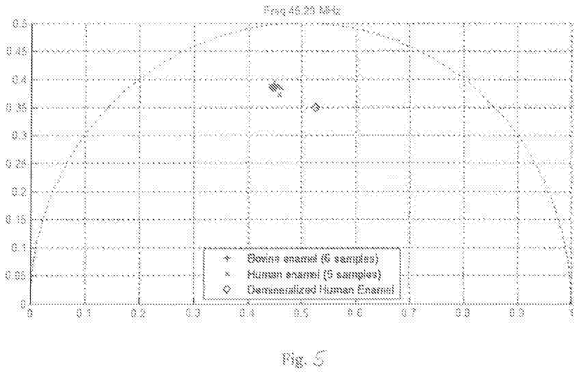

As noted above, the dental apparatus 4 can additionally be utilized to detect other tooth anomalies. For example, and with specific reference to Fig. 5, the measurement results in a polar plot of tooth enamel from different samples at 45.43MHz, e.g., bovine tooth enamel, human tooth enamel and a human enamel sample affected by demineralization is illustrated. Specifically, the detected fluorescence properties are plotted as M sin (Φ) versus M cos (Φ) (e.g., the 90° phase shifted component vs. in-phase part of the response). In accordance with the instant disclosure, a phase angle, which is an angle between the x-axis and the location at a given frequency and the distance from the origin, provides a magnitude of the response.

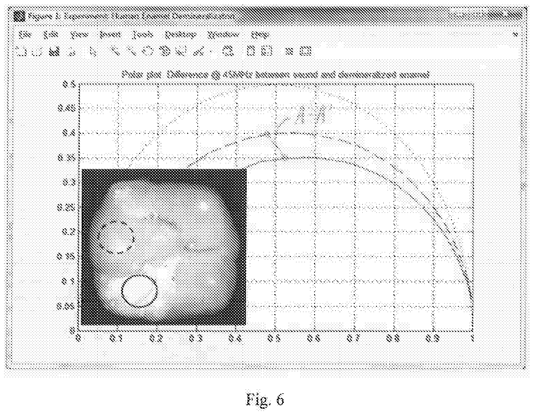

In accordance with the instant disclosure, data relating to a signal corresponding to a sound tooth (e.g., a first reference signal) and a signal corresponding to a tooth affected by demineralization (e.g., a second reference signal) was plotted as a polar plot to illustrate a difference in these signals at a particular frequency (Fig. 6). In accordance with the instant disclosure, the polar plot is utilized to extract the presence of demineralized enamel from the frequency domain fluorescence data. Specifically, a plot of M sin (Φ) versus M cos (Φ) (e.g., the 90° phase shifted component vs. in-phase part of the response), where *M* is the normalized emission modulation and *Φ* is the phase delay of the fluorescence emission with respect to an excitation signal. As evident from this graph, a measurement at a single (or a limited number of modulation frequencies) is enough to detect the presence of a white spot lesion, e.g., demineralization.

Fig. 6 shows a demineralization affected tooth and a tooth with no demineralization, i.e., a tooth with a sound surface, and allows a prediction of where a tooth with partial demineralization may lie on along the line A-A'; with low demineralization coverage being near the sound tooth location. Thus, a measure of the degree of demineralization is possible, provided the end points of the line A-A' are known.

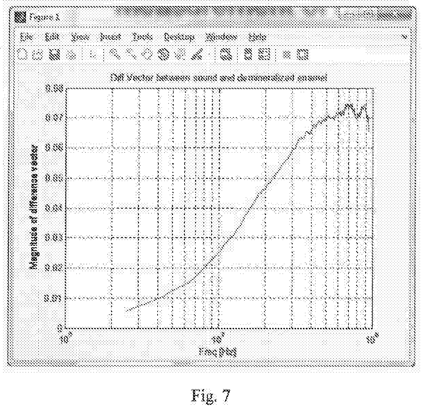

In accordance with the instant disclosure, the full frequency domain data was analyzed to determine the optimum modulation frequency. Specifically, and with reference to Fig. 7, the optimum modulation frequency was determined to be in the range from about 25 MHz to 100 MHz. With this data and the data obtained from Figs. 5 and 6, it is evident that the presence of a white spot lesion can be based on the distance from a single modulation frequency result (e.g., in, but not limited to, the range of 25 MHz to 100 MHz) to the corresponding frequency point in the sound enamel locus.

As can be appreciated, the data collected from Figs. 5-7 can also be correlated to a tooth covered with dental plaque.

Operation of system 2 is described in terms of a method 200 for detecting dental caries and/or demineralization. The dental apparatus 4 may, initially, be positioned within a mouth of a user.

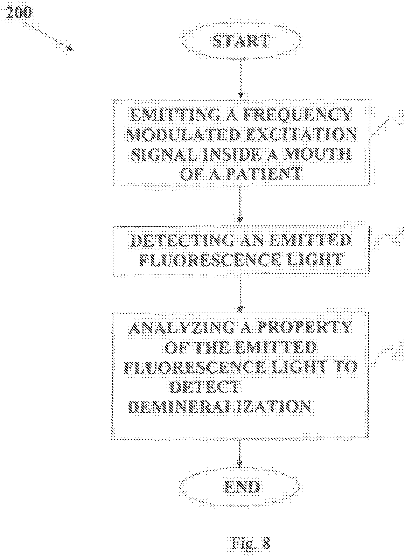

Thereafter, the plurality of bristles 14 are activated and rotated, in conventional fashion, and a frequency modulated excitation signal is emitted from LEDs 30 which causes an emitted fluorescence light to be reflected back to photodetectors 34 (see Fig. 8 at step 202); this can be accomplished on a tooth covered with toothpaste foam or free from toothpaste foam.

Thereafter, controller 20 receives an output signal from lock-in amplifier 36. The control algorithm utilizes one or more of the aforementioned transforms to calculate the phase shift (and/or amplitude) of the detected emitted fluorescence (see Fig. 8 at steps 204 and 206). The controller 20 utilizes a closed loop feedback loop to continuously monitor the presence of a phase shift of the detected emitted fluorescence to detect white spot lesions associated with demineralization at a tooth site.

In embodiments, dental apparatus 4 may be equipped with one or more indicating devices, e.g., audio, visual, etc. (not explicitly shown), that are configured to give a user an indication of dental caries and/or at the onset demineralization.

The aforementioned process repeats to continuously measure a level of dental caries and/or demineralization on the current tooth being brushed. Dental apparatus 4 can communicate the presence of dental caries and/or demineralization to the user in a wide variety of ways e.g. by illuminating the aforementioned one more LEDS that may be provided on the handle 8.

Dental apparatus 4 provides information regarding dental caries and/or demineralization in real time during brushing. Additionally, in the event that demineralization is detected, a user may begin a fluoride regiment (or other suitable regiment) to begin remineralization of a tooth, which, in turn, can restore the tooth with sound enamel. The dental apparatus 4 accomplishes the foregoing, without the use of the aforementioned bulky, expensive components that utilize high voltage and that are typically associated with conventional dental caries and/or demineralization apparatuses.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the patent.

## Claims

1. A dental apparatus (4), comprising:
a handle (6);
a controller (20) operably coupled to the handle (6); and
a subsystem (22) in operable communication with the controller (20) and configured to generate a frequency- or time-modulated excitation signal causing an emitted fluorescence light to return to the subsystem (22), the controller (20) being for analyzing at least one property of the emitted fluorescence light, the at least one property corresponding to a decay time of the emitted fluorescence light,
wherein the controller (20) is programmed to analyze detected emitted fluorescence light to determine, based on the property of the emitted fluorescence light that plaque-emitted fluorescence light decays faster than tooth-emitted fluorescence light, whether the emitted fluorescence light is indicative of a plaque-emitted fluorescence light decay time or tooth-emitted fluorescence light decay time, to detect the presence of dental plaque.

2. The dental apparatus (4) according to claim 1, wherein the frequency modulated excitation signal is provided on one of a single frequency and multiple frequencies.

3. The dental apparatus (4) according to claim 1, wherein the subsystem (22) includes at least one of a light emitting diode (30), a laser diode, a filter (32), a photodetector (34), an imaging sensor, an amplifier (36), an oscillator (38), a mixer (42), a beam-splitter (40) and an analog to digital converter (46).

4. The dental apparatus (4) according to claim 3, wherein the filter (32) is an excitation cleanup filter, the oscillator (38) is a single or multi-frequency modulated oscillator and the beam-splitter (40) is a dichroic beam-splitter.

5. The dental apparatus (4) according to claim 1, wherein the subsystem (22) is configured to detect emitted fluorescence light in a frequency that ranges from about 10 Hz to about 10 GHz.

6. The dental apparatus (4) according to claim 1, further including a calibration module (44) that is in operable communication with the controller (20) for calibrating out phase delays and frequency dependent gains associated with a tooth of a patient.

7. The dental apparatus (4) according to claim 1, wherein a battery (8) is housed within the handle (6) and is configured to supply power to the dental apparatus (4) including a motor (10) that is housed within the handle (6) and the subsystem (22), which is housed in a shaft (18) that extends distally from handle (6).

8. The dental apparatus (4) according to claim 1, further including a toothbrush assembly (16) that is configured to releasably couple to the shaft (18) for at least brushing teeth and removing the dental plaque.

9. The dental apparatus (4) according to claim 8, wherein a window (24) is positioned on the toothbrush assembly (16) adjacent a plurality of bristles (14) provided thereon and aligns with the subsystem (22) disposed on the shaft (18) such that the excitation signal and the emitted fluorescence light are passable through the window (24).

10. The dental apparatus (4) according to claim 1, wherein the at least one property of the emitted fluorescence light corresponds to a faster decay time and lower phase shift associated with the emitted fluorescence light and an amplitude of the emitted fluorescence light.

11. The dental apparatus (4) according to claim 10, wherein the controller (20) includes at least one control algorithm configured to analyze the phase shift of the emitted fluorescence light and the amplitude of the emitted fluorescence light utilizing one of frequency domain analysis and time domain analysis methods.

12. The dental apparatus (4) according to claim 1, wherein the handle (6) includes a shaft (18) extending distally therefrom, and a battery (8), motor (10) and the controller (20) being housed therein, the dental apparatus (4) further comprising a toothbrush assembly (16) configured to removably couple to the shaft (18).

13. The dental apparatus (4) according to claim 12, wherein the frequency modulated excitation signal is provided on one of a single frequency and multiple frequencies.

14. The dental apparatus (4) according to claim 12, wherein the subsystem (22) includes at least one of a light emitting diode (30), a laser diode, a filter (32), a photodetector (34), an imaging sensor, an amplifier (36), an oscillator (38), a mixer (42), a beam-splitter (40) and an analog to digital converter (46).

15. The dental apparatus (4) according to claim 14, wherein the filter (32) is an excitation cleanup filter, the oscillator (38) is a single or multi-frequency modulated oscillator and the beam-splitter (40) is a dichroic beam-splitter.

16. The dental apparatus (4) according to claim 12, wherein the subsystem is configured to detect emitted fluorescence light in a frequency that ranges from about 10Hz to about 10GHz.

17. The dental apparatus (4) according to claim 12, further including a calibration module (44) that is in operable communication with the controller (20) for calibrating out phase delays and frequency dependent gains associated with a tooth of a patient.

18. The dental apparatus (4) according to claim 12, wherein a window (24) is positioned on the toothbrush assembly (16) adjacent a plurality of bristles (14) provided thereon and aligns with the subsystem (22) disposed on the shaft (18) such that the excitation signal and the emitted fluorescence light are passable through the window (24).

19. The dental apparatus (4) according to claim 12, wherein the at least one property of the emitted fluorescence light corresponds to a faster decay time and lower phase shift associated with the emitted fluorescence light and an amplitude associated with the emitted fluorescence light.

20. The dental apparatus (4) according to claim 19, wherein the controller (20) includes at least one control algorithm configured to analyze the phase shift of the emitted fluorescence light and the amplitude of the emitted fluorescence light utilizing one of frequency domain analysis and time domain analysis methods.

21. A method (100) for detecting dental plaque at a site on a tooth, the method comprising:
emitting a frequency- or time-modulated excitation signal inside a mouth of a patient;
detecting an emitted fluorescence light; and
analyzing at least one property of the emitted fluorescence light; and
analyzing detected emitted fluorescence light to determine, based on the property of the emitted fluorescence light that plaque-emitted fluorescence light decays faster than tooth-emitted fluorescence light, whether the emitted fluorescence light is indicative of a plaque emitted fluorescence light decay time or tooth emitted fluorescence light decay time, to detect the presence of dental plaque.

22. The method (100) according to claim 21, including frequency modulating the excitation signal on one of a single frequency and multiple frequencies.

23. The method (100) according to claim 21, including detecting the emitted fluorescence light in a frequency that ranges from about 10 Hz to about 10 GHz.

24. The method (100) according to claim 21, wherein analyzing further includes analyzing one of a phase shift and amplitude of the emitted fluorescence light.

25. The method (100) according to claim 24, including utilizing frequency domain analysis methods to analyze the phase shift and amplitude of the emitted fluorescence light.

26. The method (100) according to claim 21, including providing a dental apparatus (4) for emitting, detecting and analyzing, and providing the dental apparatus (4) with a handle (6) including a battery (8) that is configured to supply power to the dental apparatus (4) including a motor (10) that is housed within the handle (6), a controller (20) and subsystem (22), which is housed in a shaft (18) that extends distally from handle (6).

27. The method (100) according to claim 26, including providing the subsystem (22) with at least one of a light emitting diode (30), a laser diode, a filter (32), a photodetector (34), an imaging sensor, an amplifier (36), an oscillator (38), a mixer (42), a beam-splitter (40) and an analog to digital converter (46).

28. The method (100) according to claim 27, including utilizing an optical excitation cleanup filter, a single or multi-frequency modulated oscillator and a dichroic beam-splitter.

29. The method (100) according to claim 21, including calibrating out phase delays and frequency dependent gains associated with the tooth via a calibration module (44) that is in operable communication with the controller (20).

## Patentansprüche

1. Dentale Vorrichtung (4), umfassend:
einen Griff (6);
eine Steuerung (20), die funktionsfähig mit dem Griff (6) gekoppelt ist; und
ein Teilsystem (22) in betriebsfähiger Verbindung mit der Steuerung (20) und das konfiguriert ist, um ein frequenz- oder zeitmoduliertes Erregungssignal zu erzeugen, das bewirkt, dass ein emittiertes Fluoreszenzlicht zu dem Teilsystem (22) zurückkehrt,
wobei die Steuerung (20) zum Analysieren mindestens einer Eigenschaft des emittierten Fluoreszenzlichts ist, wobei die mindestens eine Eigenschaft einer Abklingzeit des emittierten Fluoreszenzlichts entspricht,
wobei die Steuerung (20) programmiert ist, um erfasstes emittiertes Fluoreszenzlicht zu analysieren, um basierend auf der Eigenschaft des emittierten Fluoreszenzlichts, dass von Zahnbelag emittiertes Fluoreszenzlicht schneller abklingt als von Zähnen emittiertes Fluoreszenzlicht, zu bestimmen, ob das emittierte Fluoreszenzlicht indikativ für eine Abklingzeit von von Zahnbelag emittiertem Fluoreszenzlicht oder eine Abklingzeit von von Zähnen emittiertem Fluoreszenzlicht ist, um das Vorhandensein von Zahnbelag zu erfassen.

2. Dentale Vorrichtung (4) nach Anspruch 1, wobei das frequenzmodulierte Erregungssignal auf einer von einer einzelnen Frequenz und vielfachen Frequenzen bereitgestellt wird.

3. Dentale Vorrichtung (4) nach Anspruch 1, wobei das Teilsystem (22) mindestens eine lichtemittierende Diode (30), eine Laserdiode, einen Filter (32), einen Fotodetektor (34), einen Bildsensor, einen Verstärker (36), einen Oszillator (38), einen Mischer (42), einen Strahlteiler (40) und einen Analog-Digital-Wandler (46) beinhaltet.

4. Dentale Vorrichtung (4) nach Anspruch 3, wobei der Filter (32) ein Erregungsreinigungsfilter ist, der Oszillator (38) ein ein- oder mehrfrequenzmodulierter Oszillator ist und der Strahlteiler (40) ein dichroitischer Strahlteiler ist.

5. Dentale Vorrichtung (4) nach Anspruch 1, wobei das Teilsystem (22) konfiguriert ist, um emittiertes Fluoreszenzlicht mit einer Frequenz in einem Bereich von etwa 10 Hz bis etwa 10 GHz zu erfassen.

6. Dentale Vorrichtung (4) nach Anspruch 1, das ferner ein Kalibrierungsmodul (44) beinhaltet, das in funktionsfähiger Verbindung mit der Steuerung (20) ist, um Phasenverzögerungen und frequenzabhängige Verstärkungen zu kalibrieren, die mit einem Zahn eines Patienten assoziiert sind.

7. Dentale Vorrichtung (4) nach Anspruch 1, wobei eine Batterie (8) in dem Griff (6) untergebracht und konfiguriert ist, um die dentale Vorrichtung (4), die einen Motor (10), der in dem Griff (6) untergebracht ist, und das Teilsystem (22) beinhaltet, das in einem Schaft (18) untergebracht ist, der sich distal von dem Griff (6) erstreckt, mit Strom zu versorgen.

8. Dentale Vorrichtung (4) nach Anspruch 1, die ferner eine Zahnbürstenanordnung (16) beinhaltet, die konfiguriert ist, um lösbar mit dem Schaft (18) gekoppelt zu werden, um mindestens die Zähne zu putzen und den Zahnbelag zu entfernen.

9. Dentale Vorrichtung (4) nach Anspruch 8, wobei ein Fenster (24) an der Zahnbürstenanordnung (16) angrenzend an eine Vielzahl von daran bereitgestellten Borsten (14) positioniert ist und mit dem Teilsystem (22), das an dem Schaft (18) angeordnet ist, ausgerichtet ist, sodass das Erregungssignal und das emittierte Fluoreszenzlicht durch das Fenster (24) verlaufen können.

10. Dentale Vorrichtung (4) nach Anspruch 1, wobei die mindestens eine Eigenschaft des emittierten Fluoreszenzlichts einer schnelleren Abklingzeit und einer geringeren Phasenverschiebung in Verbindung mit dem emittierten Fluoreszenzlicht und einer Amplitude des emittierten Fluoreszenzlichts entspricht.

11. Dentale Vorrichtung (4) nach Anspruch 10, wobei die Steuerung (20) mindestens einen Steueralgorithmus beinhaltet, der konfiguriert ist, um die Phasenverschiebung des emittierten Fluoreszenzlichts und die Amplitude des emittierten Fluoreszenzlichts unter Verwendung eines der Verfahren Frequenzbereichsanalyse und Zeitbereichsanalyse zu analysieren.

12. Dentale Vorrichtung (4) nach Anspruch 1, wobei der Griff (6) einen Schaft (18) beinhaltet, der sich distal davon erstreckt, und eine Batterie (8), ein Motor (10) und die Steuerung (20) darin untergebracht sind, die dentale Vorrichtung (4) ferner umfassend eine Zahnbürstenanordnung (16), die konfiguriert ist, um abnehmbar mit dem Schaft (18) gekoppelt zu sein.

13. Dentale Vorrichtung (4) nach Anspruch 12, wobei das frequenzmodulierte Erregungssignal auf einer von einer einzelnen Frequenz und vielfachen Frequenzen bereitgestellt wird.

14. Dentale Vorrichtung (4) nach Anspruch 12, wobei das Teilsystem (22) mindestens eine lichtemittierende Diode (30), eine Laserdiode, einen Filter (32), einen Fotodetektor (34), einen Bildsensor, einen Verstärker (36), einen Oszillator (38), einen Mischer (42), einen Strahlteiler (40) und einen Analog-Digital-Wandler (46) beinhaltet.

15. Dentale Vorrichtung (4) nach Anspruch 14, wobei der Filter (32) ein Erregungsreinigungsfilter ist, der Oszillator (38) ein ein- oder mehrfrequenzmodulierter Oszillator ist und der Strahlteiler (40) ein dichroitischer Strahlteiler ist.

16. Dentale Vorrichtung (4) nach Anspruch 12, wobei das Teilsystem konfiguriert ist, um emittiertes Fluoreszenzlicht mit einer Frequenz in einem Bereich von etwa 10 Hz bis etwa 10 GHz zu erfassen.

17. Dentale Vorrichtung (4) nach Anspruch 12, das ferner ein Kalibrierungsmodul (44) beinhaltet, das in funktionsfähiger Verbindung mit der Steuerung (20) ist, um Phasenverzögerungen und frequenzabhängige Verstärkungen zu kalibrieren, die mit einem Zahn eines Patienten assoziiert sind.

18. Dentale Vorrichtung (4) nach Anspruch 12, wobei ein Fenster (24) an der Zahnbürstenanordnung (16) angrenzend an eine Vielzahl von daran bereitgestellten Borsten (14) positioniert ist und mit dem Teilsystem (22), das an dem Schaft (18) angeordnet ist, ausgerichtet ist, sodass das Erregungssignal und das emittierte Fluoreszenzlicht durch das Fenster (24) verlaufen können.

19. Dentale Vorrichtung (4) nach Anspruch 12, wobei die mindestens eine Eigenschaft des emittierten Fluoreszenzlichts einer schnelleren Abklingzeit und einer geringeren Phasenverschiebung in Verbindung mit dem emittierten Fluoreszenzlicht und einer Amplitude, die mit dem emittierten Fluoreszenzlicht assoziiert ist, entspricht.

20. Dentale Vorrichtung (4) nach Anspruch 19, wobei die Steuerung (20) mindestens einen Steueralgorithmus beinhaltet, der konfiguriert ist, um die Phasenverschiebung des emittierten Fluoreszenzlichts und die Amplitude des emittierten Fluoreszenzlichts unter Verwendung eines der Verfahren Frequenzbereichsanalyse und Zeitbereichsanalyse zu analysieren.

21. Verfahren (100) zum Erfassen von Zahnbelag an einer Stelle auf einem Zahn, das Verfahren umfassend:
Emittieren eines frequenz- oder zeitmodulierten Erregungssignals in den Mund eines Patienten;
Erfassen eines emittierten Fluoreszenzlichts; und
Analysieren mindestens einer Eigenschaft des emittierten Fluoreszenzlichts; und
Analysieren des erfassten emittierten Fluoreszenzlichts, um erfasstes emittiertes Fluoreszenzlicht zu analysieren, um basierend auf der Eigenschaft des emittierten Fluoreszenzlichts, dass von Zahnbelag emittiertes Fluoreszenzlicht schneller abklingt als von Zähnen emittiertes Fluoreszenzlicht, zu bestimmen, ob das emittierte Fluoreszenzlicht indikativ für eine Abklingzeit von von Zahnbelag emittiertem Fluoreszenzlicht oder eine Abklingzeit von von Zähnen emittiertem Fluoreszenzlicht ist, um das Vorhandensein von Zahnbelag zu erfassen.

22. Verfahren (100) nach Anspruch 21, das ein Frequenzmodulieren des Erregungssignals auf einer von einer einzelnen Frequenz und vielfachen Frequenzen beinhaltet.

23. Verfahren (100) nach Anspruch 21, das ein Erfassen des emittierten Fluoreszenzlichts mit einer Frequenz in einem Bereich von etwa 10 Hz bis etwa 10 GHz beinhaltet.

24. Verfahren (100) nach Anspruch 21, wobei das Analysieren ferner ein Analysieren einer von einer Phasenverschiebung oder einer Amplitude des emittierten Fluoreszenzlichts beinhaltet.

25. Verfahren (100) nach Anspruch 24, das ein Verwenden von Frequenzbereichsanalyse-Verfahren zum Analysieren der Phasenverschiebung und Amplitude des emittierten Fluoreszenzlichts beinhaltet.

26. Verfahren (100) nach Anspruch 21, das ein Bereitstellen einer dentalen Vorrichtung (4) zum Emittieren, Erfassen und Analysieren, und ein Versehen der dentalen Vorrichtung (4) mit einem Griff (6), der eine Batterie (8) beinhaltet, die konfiguriert ist, um die dentale Vorrichtung (4), die einen Motors (10), der in dem Griff (6) untergebracht ist, eine Steuerung (20) und ein Teilsystems (22) beinhaltet, das in einem Schaft (18) untergebracht ist, der sich distal von dem Griff (6) erstreckt, mit Strom zu versorgen, beinhaltet.

27. Verfahren (100) nach Anspruch 26, das ein Versehen des Teilsystems (22) mit mindestens einer lichtemittierenden Diode (30), einer Laserdiode, einem Filter (32), einem Photodetektor (34), einem Bildsensor, einem Verstärker (36), einem Oszillator (38), einem Mischer (42), einem Strahlteiler (40) und einem Analog-Digital-Wandler (46) beinhaltet.

28. Verfahren (100) nach Anspruch 27, das ein Verwenden eines optischen Erregungsbereinigungsfilters, eines ein- oder mehrfrequenzmodulierten Oszillators und eines dichroitischen Strahlteilers beinhaltet.

29. Verfahren (100) nach Anspruch 21, das ein Kalibrieren von Phasenverzögerungen und frequenzabhängigen Verstärkungen, die mit dem Zahn assoziiert sind, über ein Kalibrierungsmodul (44), das in funktionsfähiger Verbindung mit der Steuerung (20) ist, beinhaltet.

## Revendications

1. Appareil dentaire (4), comprenant:
une poignée (6);
un dispositif de commande (20) couplé de manière fonctionnelle à la poignée (6); et
un sous-système (22) en communication opérationnelle avec le dispositif de commande (20) et configuré pour générer un signal d'excitation modulé en fréquence ou
dans le temps provoquant le retour d'une lumière de fluorescence émise vers le sous-système (22), le dispositif de commande (20) étant destiné à analyser au moins une propriété de la lumière de fluorescence émise, l'au moins une propriété correspondant à un temps de désintégration de la lumière de fluorescence émise,
dans lequel le dispositif de commande (20) est programmé pour analyser la lumière de fluorescence émise détectée pour déterminer, sur la base de la propriété de la lumière de fluorescence émise selon laquelle la lumière de fluorescence émise par la plaque se désintègre plus rapidement que la lumière de fluorescence émise par les dents, que la lumière de fluorescence émise soit indicative d'un temps de désintégration de lumière de fluorescence émise par la plaque ou d'un temps de désintégration de lumière de fluorescence émise par les dents, pour détecter la présence de plaque dentaire.

2. Appareil dentaire (4) selon la revendication 1, dans lequel le signal d'excitation modulé en fréquence est fourni sur une parmi une seule fréquence et de multiples fréquences.

3. Appareil dentaire (4) selon la revendication 1, dans lequel le sous-système (22) inclut au moins un parmi une diode électroluminescente (30), une diode laser, un filtre (32), un photodétecteur (34), un capteur d'imagerie, un amplificateur (36), un oscillateur (38), un mélangeur (42), un séparateur de faisceau (40) et un convertisseur analogique-numérique (46) .

4. Appareil dentaire (4) selon la revendication 3, dans lequel le filtre (32) est un filtre de nettoyage d'excitation, l'oscillateur (38) est un oscillateur modulé à une ou plusieurs fréquences et le séparateur de faisceau (40) est un séparateur de faisceau dichroïque.

5. Appareil dentaire (4) selon la revendication 1, dans lequel le sous-système (22) est configuré pour détecter de la lumière de fluorescence émise à une fréquence qui s'étend d'environ 10 Hz à environ 10 GHz.

6. Appareil dentaire (4) selon la revendication 1, incluant en outre un module d'étalonnage (44) qui est en communication opérationnelle avec le dispositif de commande (20) pour étalonner des retards de phase et gains dépendant de la fréquence associés à une dent d'un patient.

7. Appareil dentaire (4) selon la revendication 1, dans lequel une batterie (8) est logée au sein de la poignée (6) et est configurée pour alimenter l'appareil dentaire (4) incluant un moteur (10) qui est logé au sein de la poignée (6) et le sous-système (22), qui est logé dans un arbre (18) qui s'étend distalement à partir de la poignée (6).

8. Appareil dentaire (4) selon la revendication 1, incluant en outre un assemblage de brosse à dents (16) qui est configuré pour se coupler de manière amovible à l'arbre (18) pour au moins se brosser les dents et enlever la plaque dentaire.

9. Appareil dentaire (4) selon la revendication 8, dans lequel une fenêtre (24) est positionnée sur l'assemblage de brosse à dents (16) adjacente à une pluralité de poils (14) fournis sur celui-ci et s'aligne avec le sous-système (22) disposé sur l'arbre (18) de sorte que le signal d'excitation et la lumière de fluorescence émise peuvent passer à travers la fenêtre (24).

10. Appareil dentaire (4) selon la revendication 1, dans lequel l'au moins une propriété de la lumière de fluorescence émise correspond à un temps de désintégration plus rapide et à un déphasage inférieur associé à la lumière de fluorescence émise et à une amplitude de la lumière de fluorescence émise.

11. Appareil dentaire (4) selon la revendication 10, dans lequel le dispositif de commande (20) inclut au moins un algorithme de commande configuré pour analyser le déphasage de la lumière de fluorescence émise et l'amplitude de la lumière de fluorescence émise en utilisant une parmi des méthodes d'analyse de domaine de fréquence et d'analyse temporelle.

12. Appareil dentaire (4) selon la revendication 1, dans lequel la poignée (6) inclut un arbre (18) s'étendant distalement à partir de celle-ci, et une batterie (8), un moteur (10) et le dispositif de commande (20) étant logés à l'intérieur, l'appareil dentaire appareil (4) comprenant en outre un assemblage de brosse à dents (16) configuré pour se coupler de manière amovible à l'arbre (18).

13. Appareil dentaire (4) selon la revendication 12, dans lequel le signal d'excitation modulé en fréquence est fourni sur une parmi une seule fréquence et de multiples fréquences.

14. Appareil dentaire (4) selon la revendication 12, dans lequel le sous-système (22) inclut au moins un parmi une diode électroluminescente (30), une diode laser, un filtre (32), un photodétecteur (34), un capteur d'imagerie, un amplificateur (36), un oscillateur (38), un mélangeur (42), un séparateur de faisceau (40) et un convertisseur analogique-numérique (46) .

15. Appareil dentaire (4) selon la revendication 14, dans lequel le filtre (32) est un filtre de nettoyage d'excitation, l'oscillateur (38) est un oscillateur modulé à une ou plusieurs fréquences et le séparateur de faisceau (40) est un séparateur de faisceau dichroïque.

16. Appareil dentaire (4) selon la revendication 12, dans lequel le sous-système est configuré pour détecter de la lumière de fluorescence émise à une fréquence qui s'étend d'environ 10Hz à environ 10GHz.

17. Appareil dentaire (4) selon la revendication 12, incluant en outre un module d'étalonnage (44) qui est en communication opérationnelle avec le dispositif de commande (20) pour étalonner des retards de phase et gains dépendant de la fréquence associés à une dent d'un patient.

18. Appareil dentaire (4) selon la revendication 12, dans lequel une fenêtre (24) est positionnée sur l'assemblage de brosse à dents (16) adjacente à une pluralité de poils (14) fournis sur celui-ci et s'aligne avec le sous-système (22) disposé sur l'arbre (18) de sorte que le signal d'excitation et la lumière de fluorescence émise peuvent passer à travers la fenêtre (24).

19. Appareil dentaire (4) selon la revendication 12, dans lequel l'au moins une propriété de la lumière de fluorescence émise correspond à un temps de désintégration plus rapide et à un déphasage inférieur associé à la lumière de fluorescence émise et à une amplitude associée à la lumière de fluorescence émise.

20. Appareil dentaire (4) selon la revendication 19, dans lequel le dispositif de commande (20) inclut au moins un algorithme de commande configuré pour analyser le déphasage de la lumière de fluorescence émise et l'amplitude de la lumière de fluorescence émise en utilisant une parmi des méthodes d'analyse de domaine de fréquence et d'analyse temporelle.

21. Procédé (100) pour détecter de la plaque dentaire au niveau d'un site sur une dent, le procédé consistant à: émettre un signal d'excitation modulé en fréquence ou dans le temps à l'intérieur d'une bouche d'un patient; détecter une lumière de fluorescence émise; et analyser au moins une propriété de la lumière de fluorescence émise; et analyser de la lumière de fluorescence émise détectée pour déterminer, sur la base de la propriété de la lumière de fluorescence émise selon laquelle de la lumière de fluorescence émise par la plaque se désintègre plus rapidement que de la lumière de fluorescence émise par les dents, que la lumière de fluorescence émise soit indicative d'un temps de désintégration de lumière de fluorescence émise par la plaque ou un temps de désintégration de lumière de fluorescence émise par les dents, pour détecter la présence de plaque dentaire.

22. Procédé (100) selon la revendication 21, consistant à moduler en fréquence le signal d'excitation sur une parmi une seule fréquence et de multiples fréquences.

23. Procédé (100) selon la revendication 21, consistant à détecter la lumière de fluorescence émise à une fréquence qui s'étend d'environ 10 Hz à environ 10 GHz.

24. Procédé (100) selon la revendication 21, dans lequel l'analyse inclut en outre l'analyse d'un déphasage et d'une amplitude de la lumière de fluorescence émise.

25. Procédé (100) selon la revendication 24, consistant à utiliser des procédés d'analyse de domaine de fréquence pour analyser le déphasage et l'amplitude de la lumière de fluorescence émise.

26. Procédé (100) selon la revendication 21, consistant à fournir un appareil dentaire (4) pour émettre, détecter et analyser, et munir l'appareil dentaire (4) d'une poignée (6) incluant une batterie (8) qui est configurée pour alimenter l'appareil dentaire (4) incluant un moteur (10) qui est logé au sein de la poignée (6), un dispositif de commande (20) et un sous-système (22), qui est logé dans un arbre (18) qui s'étend distalement à partir de la poignée (6) .

27. Procédé (100) selon la revendication 26, consistant à fournir au sous-système (22) au moins un parmi une diode électroluminescente (30), une diode laser, un filtre (32), un photodétecteur (34), un capteur d'imagerie, un amplificateur (36), un oscillateur (38), un mélangeur (42), un séparateur de faisceau (40) et un convertisseur analogique-numérique (46) .

28. Procédé (100) selon la revendication 27, consistant à utiliser un filtre de nettoyage d'excitation optique, un oscillateur modulé à une ou plusieurs fréquences et un séparateur de faisceau dichroïque.

29. Procédé (100) selon la revendication 21, consistant à étalonner des retards de phase et des gains dépendant de la fréquence associés à la dent par l'intermédiaire d'un module d'étalonnage (44) qui est en communication fonctionnelle avec le dispositif de commande (20).
